# EUROPÄISCHE PATENTSCHRIFT

(11) **EP 3 483 255 B1**
(45) Veröffentlichungstag und Bekanntmachung des Hinweises auf die Patenterteilung: **15.04.2020**
(21) Anmeldenummer: 18203023.9
(22) Anmeldetag: 29.10.2018
(51) Int. Cl.: C12M 1/107, C12M 1/00, C12M 3/00

(54) **VERFAHREN ZUM SCHÜTZEN EINES GÄRBEHÄLTERS EINER BIOGASANLAGE SOWIE GÄRBEHÄLTER EINER BIOGASANLAGE**
METHOD FOR PROTECTING A COOKING CONTAINER OF A BIOGAS SYSTEM AND COOKING CONTAINER OF A BIOGAS SYSTEM
PROCÉDÉ DE PROTECTION D'UN RÉCIPIENT DE FERMENTATION UNE INSTALLATION BIO-GAZ AINSI QUE RÉCIPIENT DE FERMENTATION D'UNE INSTALLATION BIO-GAZ

(30) Priorität: 13.11.2017 DE 102017126628
(43) Veröffentlichungstag der Anmeldung: 15.05.2019
(73) Patentinhaber: AGROTEL GmbH, 94152 Neuhaus/Inn (DE)
(72) Erfinder: LANER, Cyriak, 94152 Neuhaus/Inn (DE)
(74) Vertreter: Prinz & Partner mbB

(56) Entgegenhaltungen:
- EP-A1- 1 867 712
- WO-A1-2013/110821
- DE-A1- 3 731 617
- DE-A1- 10 354 598
- DE-A1-102004 015 694
- DE-A1-102005 061 039
- DE-U1-202006 014 149

## Beschreibung

Die Erfindung betrifft ein Verfahren zum Schützen eines Gärbehälters einer Biogasanlage sowie einen Gärbehälter einer Biogasanlage.

Der Gärbehälter einer Biogasanlage besteht üblicherweise aus Beton. Dabei ist der Beton, wenn er nicht durch geeignete Maßnahmen geschützt ist, während des Betriebs der Biogasanlagen der sogenannten biogenen Schwefelsäure-Korrosion (BSK) ausgesetzt. Diese entsteht, wenn durch mikrobiologische Prozesse Schwefelsäure (H₂SO₄) entsteht, die die Oberfläche des Betons angreift. Dies betrifft insbesondere die sogenannte Gaswechselzone des Gärbehälters, also den Bereich, der nicht dauerhaft mit Flüssigkeit bedeckt ist.

Als Folge der BSK wird der Beton während des Betriebs porös und kann zerstört werden. Die Oberfläche kann dann eine Waschbeton-ähnliche Struktur aufweisen. Bei stärkerer Schädigung sind Risse und Ausbrechungen zu sehen. Im schlimmsten Fall kommt es zum Einsturz des Gärbehälters.

Abhängig von der Schwere der Beschädigung des Betons sind kostenintensive Sanierungsmaßnahmen nötig. Zusätzlich zu diesen direkten Kosten kommt es zu indirekten Kosten in der Form von Einnahmeausfällen, da die Biogasanlage für die Dauer der Sanierung nicht betrieben und dementsprechend keine Energie liefern kann.

Gemäß Stand der Technik, DE 10 2004 015694 und EP 1 867 712, ist es fachüblich, die Gärbehälterinnenwandung bei Gärbehälterherstellung im Vorfeld bereits mit einer Folie zu schützen. Die Verbundmatten sind an der Betonwand in dem noch nassen und fließfähigem Beton als Flechtgewebe der Verbundmatte mit dem Beton verbunden.

Die Aufgabe der Erfindung besteht darin, eine aufwendige Sanierung eines beschädigten Gärbehälters einer Biogasanlage zu verhindern.

Zur Lösung dieser Aufgabe ist erfindungsgemäß ein Verfahren zum Schützen eines Gärbehälters einer Biogasanlage mittels der folgenden Schritte vorgesehen: Auf die Innenseite der Wand des Gärbehälters wird mindestens eine in Umfangsrichtung umlaufende Bahn einer Schutzfolie angeordnet, die mittels mehrerer Verankerungselemente an der Wand befestigt wird. Auf die Schutzfolie wird im Bereich der Verankerungselemente und im Bereich des Stoßes zwischen Anfang und Ende der Schutzfolie mediendicht eine Abdeckung aufgeschweißt. Die genannte Aufgabe wird erfindungsgemäß auch gelöst durch einen Gärbehälter für eine Biogasanlage, mit einer Wand aus Beton, die zumindest in einer Gaswechselzone durch aggressive Medien vorgeschädigt ist, und einer Schutzfolie, die im Innenraum des Gärbehälters an der Wand angebracht ist, wobei die Schutzfolie an ihrem oberen Rand mit einem Kronenschutz versehen ist, der sich über den oberen Rand der Wand nach außen erstreckt, wobei eine Abdichtung zwischen dem unterem Rand der Schutzfolie und der Wand vorgesehen ist und wobei mehrere Verankerungselemente vorgesehen sind, mit denen die Schutzfolie an der Wand des Gärbehälters verankert ist, wobei die Verankerungselemente mediendicht von einer Abdeckung abgedeckt sind, die mit der Schutzfolie verschweißt ist.

Die Erfindung beruht auf dem Grundgedanken, einen durch BSK geschädigten Gärbehälter nicht als solches zu sanieren, sondern durch die Schutzfolie diejenigen Bereiche der Wand, die der BSK besonders stark ausgesetzt sind, zu schützen. Hierdurch wird eine weitere Schädigung des Betons verhindert. Unter der Annahme, dass der Gärbehälter noch nicht so stark beschädigt ist, dass seine Standsicherheit beeinträchtigt ist, ist es lediglich erforderlich, die Schutzfolie anzubringen; es sind keine Sanierungsarbeiten nötig. Die Schutzfolie kann innerhalb sehr kurzer Zeit angebracht werden, insbesondere wenn sie vorab individuell für den entsprechenden Gärbehälter gefertigt wurde. Dadurch sind die Stillstandszeiten minimiert.

Besonders vorteilhaft ist, dass die Schutzfolie meist nur entlang des oberen Randes der Mauer angebracht werden muss, da es (jedenfalls bei Gärbehältern mit einem nahezu konstanten Füllstand) nur dort zur BSK kommt. Daher ist es oft ausreichend, lediglich eine einzige Bahn der Schutzfolie anzubringen, die eine Breite von 1 m oder 2 m haben kann. Nur wenn die Mauer über eine größere Höhe geschädigt ist, wie dies beispielsweise in einem Nachgärbehälter der Fall ist, dessen Füllhöhe stark schwankt, kann ein Bereich mit einer Höhe von bis zu 4 m bis 8 m mit der Schutzfolie abgedeckt werden. In diesem Fall können beispielsweise zwei Bahnen der Schutzfolie in Umfangsrichtung übereinander angebracht werden, und die Fuge zwischen den beiden Bahnen wird mit einer Abdeckung mediendicht versiegelt.

Zwischen dem unteren Rand der Schutzfolie und der Wand kann mit einer elastischen Dichtmasse abgedichtet werden, beispielsweise einem 1-Komponenten-Polyurethan, das mit der Luftfeuchtigkeit zu einem Elastomer aushärtet. Die Dichtmasse wird dabei insbesondere dort zwischen der Schutzfolie und der Wand aufgebracht, wo die Schutzfolie mittels der sich entlang ihres unteren Randes erstreckenden Verankerungselemente gegen die Wand gedrückt wird.

Von besonderem Vorteil ist, wenn die Mauerkrone der Wand mittels eines Kronenschutzes abgedeckt wird, der an der Schutzfolie angebracht ist. Die Mauerkrone ist nämlich der BSK in besonderem Maße ausgesetzt, da Kondenswasser, das sich an einer Abdeckung des Gärbehälters niederschlägt, insbesondere auf die Mauerkrone tropft. Der Kronenschutz ist vorzugsweise bereits an der (oberen) Bahn der Schutzfolie angebracht, wenn diese angeliefert wird. Es ist alternativ auch möglich, dass der Kronenschutz nachträglich angebracht und mit der Schutzfolie mediendicht verbunden wird, beispielsweise verschweißt.

Wesentlich für die geringen Kosten ist erfindungsgemäß, dass vor dem Aufbringen der Schutzfolie keine Betonsanierung vorgenommen wird. Es wird lediglich mittels der Schutzfolie verhindert, dass der Beton der BSK in der Zukunft ausgesetzt ist.

Die Verankerungselemente können grundsätzlich mit jedem geeigneten Mittel an der Wand des Gärbehälters verankert werden. Besonders bevorzugt ist, dass die Verankerungselemente mittels Dübeln und Schrauben mit der Wand verbunden werden. Dies ermöglicht es, die Verankerungselemente flexibel dort anzubringen, wo sie benötigt werden.

Die Verankerungselemente können entlang des oberen Randes der Schutzfolie angeordnet und durch die Schutzfolie hindurch in der Wand verankert sein, so dass die Schutzfolie dort zuverlässig gehalten ist. Aufgrund der mediendichten Abdeckung sind die Verankerungselemente und die diese an der Wand befestigenden Schrauben etc. zuverlässig vor Korrosion geschützt.

Vorzugsweise ist auch vorgesehen, dass die Verankerungselemente entlang des unteren Randes der Schutzfolie in einem Abstand von diesem angeordnet sind. Der Abstand der Verankerungselemente vom unteren Rand der Schutzfolie ermöglicht, die mediendichte Abdeckung ober- und unterhalb der Verankerungselemente anzuschweißen, so dass diese zuverlässig vor Korrosion geschützt sind.

Um die Schutzfolie besser an der Wand zu fixieren, sind mehrere der Verankerungselemente in Umfangsrichtung voneinander beabstandet vertikal angeordnet. Dabei werden mindestens zwei vertikale Verankerungselemente verwendet, nämlich eines für den Anfang und eines für das Ende jeder Bahn der Schutzfolie. Zusätzlich können, falls gewünscht, auch weitere vertikale Verankerungselemente angebracht werden, um die Schutzfolie dort an der Wand zu verankern.

Die Schutzfolie weist gemäß der bevorzugten Ausführungsform einen Gewebeuntergrund aus Polypropylen (PP) auf. Dieser verleiht der Schutzfolie eine hohe mechanische Festigkeit, so dass die Schutzfolie nicht einreißt.

Auf dem Gewebeuntergrund ist gemäß einer bevorzugten Ausführungsform der Erfindung eine Schutzbeschichtung aus PP vorgesehen. Diese macht die Schutzfolie gasundurchlässig für und dauerhaft beständig gegen die Medien, die sich beim Betrieb der Biogasanlage innerhalb des Gärbehälters befinden.

Die Schutzbeschichtung kann eine Dicke von 1,5 mm haben, so dass sie zur hohen mechanischen Widerstandfähigkeit der Schutzfolie beiträgt.

Das verwendete Material PP zeichnet sich dadurch aus, dass es die gewünschten mechanischen und chemischen Eigenschaften über ein weites Temperaturspektrum aufweist. Weiterhin ist eine Durchfeuchtung der Schutzfolie von ihrer Rückseite her kein Problem, da sie unempfindlich gegen rückwärtige Durchfeuchtung ist.

Der Kronenschutz weist vorzugsweise eine geringere Dicke als die Schutzfolie auf. Insbesondere hat er keinen Gewebeuntergrund. Dadurch ist er flexibler und kann geringfügig gedehnt werden, so dass er nach außen über die Mauerkrone gestülpt werden kann.

Vorzugsweise ist der Kronenschutz an die Schutzfolie angeschweißt, so dass ein gas- und flüssigkeitsdichter Übergang vom Kronenschutz zur Schutzfolie gewährleistet ist.

Im Hinblick auf eine gute Korrosionsbeständigkeit bestehen die Verankerungselemente vorzugsweise aus V4A.

Die einzelnen Segmente der Schutzfolie können eine Breite von 1 m bis 2 m aufweisen. Bei einer Breite von bis zu 2 m ist es meist möglich, die Wand des Gärbehälters mit einer einzige (horizontal angeordneten) Lage von Segmenten ausreichend abzudecken, da von BSK nur die Gaswechselzone des Gärbehälters und damit nur der obere Abschnitt der Wand betroffen ist.

Die Erfindung wird nachfolgend anhand zweier Ausführungsformen beschrieben, die in den beigefügten Zeichnungen dargestellt sind. In diesen zeigen:
- Figur 1 in einer schematischen, perspektivischen Ansicht einen erfindungsgemäßen Gärbehälter gemäß einer ersten Ausführungsform;
- Figur 2 in einer schematischen Schnittansicht die Wand des Gärbehälters von Figur 1;
- Figur 3 in einer schematischen Draufsicht die Wand des Gärbehälters während der Montage der Schutzfolie;
- Figur 4 schematisch die Schutzfolie in einem Querschnitt; und
- Figur 5 in einer Ansicht entsprechend derjenigen von Figur 2 eine zweite Ausführungsform.

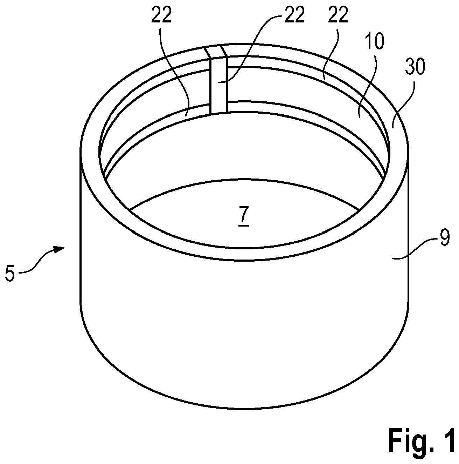

In Figur 1 ist schematisch ein Gärbehälter 5 gezeigt, der Teil einer Biogasanlage ist. Er weist einen Boden 7 auf, auf dem eine Wand 9 angeordnet ist. Im gezeigten Ausführungsbeispiel ist die Wand kreisrund.

Der Boden 7 zusammen mit der Wand 9 definiert einen Innenraum des Gärbehälters, in dem sich während des Betriebs der Biogasanlage die zu vergärende Biomasse befindet. Der besseren Übersichtlichkeit halber ist diejenige Struktur, die den Biogasbehälter auf der Oberseite verschließt (beispielsweise ein Dach oder eine Abdeckplatte), nicht dargestellt.

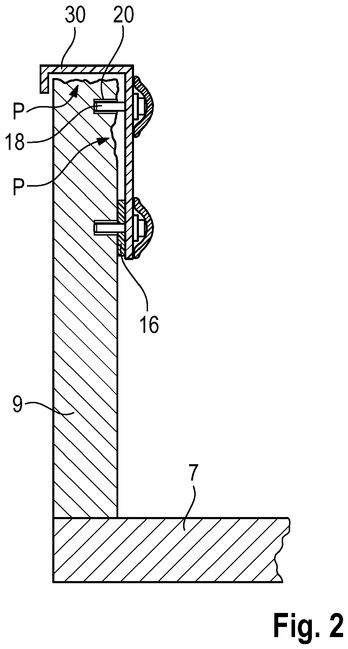

Die Wand 9 des Gärbehälters 5 besteht aus Beton, der hier im oberen Bereich der Wand 9 einer biogenen Schwefelsäure-Korrosion (BSK) ausgesetzt war. Daher ist die Wand 9 an ihrer Oberfläche beschädigt. Diese Abschnitte sind in Figur 2 mit den Pfeilen P markiert.

Biogene Schwefelsäure-Korrosion tritt insbesondere in der Gaswechselzone eines Gärbehälters auf, also oberhalb der Abschnitte der Wand, die im normalen Betrieb dauerhaft mit Flüssigkeit bedeckt sind.

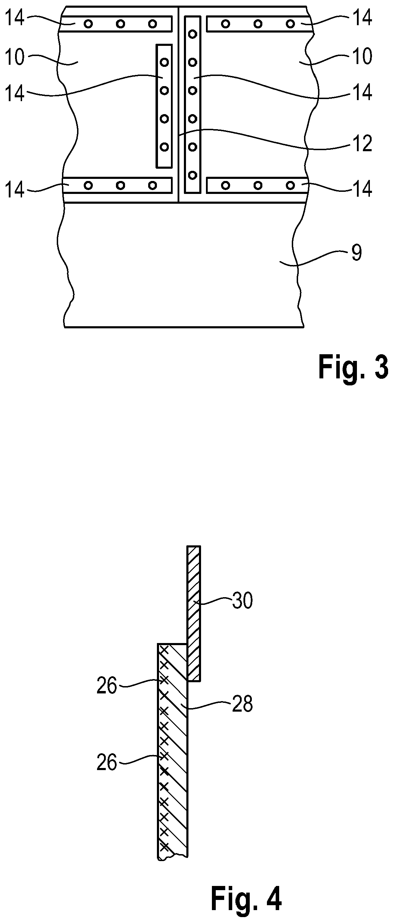

Um zu verhindern, dass der Beton der Wand 9 noch stärker geschädigt wird, ist eine Schutzfolie 10 angebracht. Diese ist in der Ausführungsform der Figuren 1 bis 3 eine horizontal umlaufende Bahn, die abgesehen von einem Stoß 12 (siehe Figur 3) durchgehend ist.

Alternativ ist auch möglich, dass die umlaufende Bahn aus mehreren miteinander verbundenen Segmenten gebildet ist.

An der Wand 9 ist die Bahn der Schutzfolie 10 mittels mehrerer Verankerungselemente 14 befestigt. Bei den Verankerungselementen 14 handelt es sich um flachen Profilstahl mit rechteckigem Querschnitt, der vorzugsweise aus V4A besteht.

Zur zuverlässigen Verankerung der Schutzfolie 10 an der Wand 9 werden mehrere Verankerungselemente 14 verwendet, die sich horizontal entlang des oberen Randes der Schutzfolie 10 und auch der Wand 9 erstrecken.

Weiterhin werden mehrere Verankerungselemente 14 verwendet, die sich horizontal entlang des unteren Randes der Schutzfolie erstrecken. Zur Verankerung werden weiterhin mehrere Verankerungselemente 14 verwendet, die sich vertikal über die Schutzfolie 10 erstrecken. Diese sind mindestens in der Nähe des Stoßes 12 angeordnet. Es können jedoch auch zusätzliche Verankerungselemente verwendet werden, die in Umfangsrichtung voneinander beabstandet angeordnet sind.

Zur Abdichtung zwischen der Schutzfolie 10 und der Wand 9 wird eine Dichtmasse 16 verwendet, die in dem Bereich angeordnet ist, in dem die unteren, horizontal angeordneten Verankerungselemente 14 die Schutzfolie gegen die Wand 9 drücken. Es kann sich hier um eine dauerelastische Dichtmasse handeln, beispielsweise aus 1-Komponenten-Polyurethan, das mit der Luftfeuchtigkeit zu einem Elastomer aushärtet.

Die Verankerungselemente 14 sind an der Wand 9 mittels Schrauben 18 und Dübeln 20 angebracht.

Sämtliche Verankerungselemente 14 sind zum Innenraum des Gärbehälters 5 mittels einer Abdeckung 22 abgedeckt, die gas- und flüssigkeitsdicht mit der Schutzfolie 10 verbunden ist. Bei den Abdeckungen 22 handelt es sich insbesondere um schmale, längliche Streifen aus einem gas- und mediendichten Material, das gut mit der Schutzfolie verbunden werden kann.

In Figur 3 sind der besseren Übersichtlichkeit halber die Abdeckungen 22 nicht dargestellt. In Figur 2 sind die beiden sich in horizontaler Richtung erstreckenden Abdeckungen 22 zu sehen, und in Figur 1 ist zusätzlich die vertikale Abdeckung 22 zu sehen, die die beiden Verankerungselemente 14 beiderseits des Stoßes 12 abdeckt.

Die Schutzfolie 10 weist einen Gewebeuntergrund 26 auf, der schematisch in Figur 4 angedeutet ist. Der Gewebeuntergrund 26 ist mit einer Schutzbeschichtung 28 versehen.

Als Material für den Gewebeuntergrund 26 und die Schutzbeschichtung 28 ist Polypropylen (PP) besonders gut geeignet. Zum einen kann die Schutzbeschichtung so auf den vorgefertigten Gewebeuntergrund 26 aufgebracht werden, dass ein unlösbarer Verbund entsteht. Außerdem ist eine Schutzbeschichtung 28 medien- und gasdicht gegenüber den Materialien, die in den Gärbehälter 5 eingefüllt werden. Die Schutzbeschichtung 28 hat insbesondere eine Dicke in der Größenordnung von 1,5 mm.

Auch die Abdeckungen 22 bestehen aus Polypropylen, so dass sie gut mit der Schutzfolie verschweißt werden können.

In Figur 4 ist auch ein Kronenschutz 30 zu sehen, der an der Schutzfolie 10 angebracht ist. Der Kronenschutz 30 erstreckt sich über die Mauerkrone der Wand 9, also über den horizontalen Abschluss der Wand 9.

Der Kronenschutz 30 besteht ebenfalls aus Polypropylen, hat jedoch eine geringere Dicke als die Schutzfolie 10. Ausreichend ist eine Dicke in der Größenordnung von 1 mm.

Vorzugsweise ist der Kronenschutz 30 mit der Schutzfolie 10 verschweißt, sodass auch hier eine mediendichte Verbindung erhalten ist.

Nachfolgend wird beschrieben, wie die Schutzfolie 10 angebracht wird.

Die angegriffene Oberfläche der Wand 9 muss nicht saniert werden, bevor die Schutzfolie 10 angebracht wird. Es ist ausreichend, sie zu reinigen, beispielsweise mittels eines Hochdruckreinigers. Anschließend wird die Bahn der Schutzfolie 10 horizontal umlaufend entlang des oberen Randes der Wand 9 angebracht, sodass diejenigen Bereiche abgedeckt sind, die der biogenen Schwefelsäure-Korrosion ausgesetzt sind. Die Schutzfolie 10 wird dabei einschließlich des Kronenschutzes 30 als aufgerollte Bahn angeliefert, die geringfügig länger als der Innenumfang der Wand 9 ist.

Die Schutzfolie 10 wird schrittweise entlang der Wand 9 mittels der Verankerungselemente 14 befestigt. Dabei wird durch die Schutzfolie 10 hindurchgebohrt und -geschraubt. Es ist also nicht nötig, vor der Anbringung der Schutzfolie 10 mittels einer Schablone oder Ähnlichem vorzubohren.

Die Verankerungselemente haben einen Dicke, die es ermöglicht, sie gut an die Kontur des Gärbehälters anzupassen. Weiterhin haben wie eine solche Länge, dass sie gut gehandhabt werden können.

Entlang des unteren Randes der Schutzfolie 10 wird dabei, bevor die unteren Verankerungselemente 14 festgeschraubt werden, die Dichtmasse 16 eingebracht, die zwischen der Schutzfolie 10 und der Wand 9 abdichtet.

Wenn die Schutzfolie 10 einmal umlaufend angebracht ist, wird der überstehende Bereich entlang des Anfangs der Schutzfolie 10 abgeschnitten, sodass der Anfang und das Ende der Schutzfolie 10 sich entlang des Stoßes 12 gegenüberliegen.

Der Kronenschutz 30 kann nach außen umgestülpt werden, sodass er sich bis auf die vertikale Außenseite der Wand 9 erstreckt. Da der Kronenschutz 30 keinerlei Gewebeuntergrund aufweist und eine geringere Wandstärke hat als die Schutzfolie, ist er ausreichend flexibel hierfür, obwohl der Außenumfang der Wand 9 länger ist als der Innenumfang und damit die Länge der Schutzfolie 10..

Die horizontal verlaufenden Verankerungselemente 14 sind in einem solchen Abstand vom oberen und vom unteren Rand der Schutzfolie 10 angebracht, dass oberhalb und unterhalb genügend Raum verbleibt, um die Abdeckungen 22 anbringen zu können. Diese werden mit der Schutzfolie 10 dicht verschweißt. Somit ist der Innenraum des Gärbehälters 5 in dem Bereich, der biogener Schwefelsäure-Korrosion ausgesetzt ist, vollständig mit einem Material ausgekleidet, das gas- und mediendicht für die sich im Gärbehälter befindenden Medien ist.

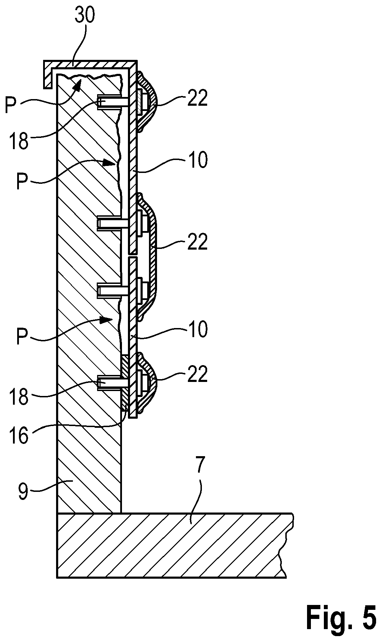

In Figur 5 ist eine zweite Ausführungsform gezeigt, die sich von der ersten Ausführungsform dadurch unterscheidet, dass sie für einen Gärbehälter 5 vorgesehen ist, dessen Wand 9 über eine größere Höhe der biogenen Schwefelsäure-Korrosion ausgesetzt ist. Daher werden hier zwei vertikal übereinander angeordnete Bahnen der Schutzfolie 10 verwendet, wobei das untere Verankerungselement 14 der oberen Schutzfolie 10 und das obere Verankerungselement 14 der unteren Schutzfolie 10 durch eine gemeinsame, breitere Abdeckung 22 gas- und mediendicht versiegelt sind.

## Patentansprüche

1. Verfahren zum Schützen eines Gärbehälters (5) einer Biogasanlage mittels der folgenden Schritte:
- auf die Innenseite der Wand (9) des Gärbehälters (5) wird mindestens eine in Umfangsrichtung umlaufende Bahn einer Schutzfolie (10) angeordnet, die mittels mehrerer Verankerungselemente (14) an der Wand (9) befestigt wird;
- auf die Schutzfolie (10) wird im Bereich der Verankerungselemente (14) und im Bereich des Stoßes (12) zwischen Anfang und Ende der Schutzfolie (10) mediendicht eine Abdeckung (22) aufgeschweißt.

2. Verfahren nach Anspruch 1, **dadurch gekennzeichnet, dass** zwei Bahnen der Schutzfolie (10) angebracht werden, wobei die Fuge zwischen den Bahnen mittels einer Abdeckung (22) mediendicht versiegelt wird.

3. Verfahren nach Anspruch 1 oder Anspruch 2, **dadurch gekennzeichnet, dass** eine Mauerkrone der Wand (9) mittels eines Kronenschutzes (30) abgedeckt wird, der an der Schutzfolie (10) angebracht ist.

4. Verfahren nach einem der vorhergehenden Ansprüche, **dadurch gekennzeichnet, dass** vor dem Aufbringen der Schutzfolie (10) keine Betonsanierung vorgenommen wird.

5. Gärbehälter (5) für eine Biogasanlage, insbesondere mittels des Verfahrens nach einem der vorhergehenden Ansprüche mit einer Schutzfolie (10) versehen, mit einer Wand (9) aus Beton, die zumindest in einer Gaswechselzone durch aggressive Medien vorgeschädigt ist, und einer Schutzfolie (10), die im Innenraum des Gärbehälters (5) an der Wand (9) angebracht ist, wobei die Schutzfolie (10) an ihrem oberen Rand mit einen Kronenschutz (30) versehen ist, der sich über den oberen Rand der Wand (9) nach außen erstreckt, wobei eine Abdichtung (16) zwischen dem unterem Rand der Schutzfolie (10) und der Wand (9) vorgesehen ist, und wobei mehrere Verankerungselemente (14) vorgesehen sind, mit denen die Schutzfolie (10) an der Wand (9) des Gärbehälters (5) verankert ist, wobei die Verankerungselemente (14) mediendicht von einer Abdeckung (22) abgedeckt sind, die mit der Schutzfolie (10) verschweißt ist.

6. Gärbehälter nach Anspruch 5, **dadurch gekennzeichnet, dass** die Schutzfolie (10) aus einer in Umfangsrichtung umlaufenden Bahn oder aus zwei in Umfangsrichtung umlaufenden Bahnen besteht.

7. Gärbehälter nach Anspruch 6, **dadurch gekennzeichnet, dass** jede Bahn eine Breite in der Größenordnung von 1 bis 2 m aufweist.

8. Gärbehälter nach einem der Ansprüche 5 bis 7, **dadurch gekennzeichnet, dass** die Verankerungselemente (14) entlang des oberen Randes der Schutzfolie (10) angeordnet und durch die Schutzfolie (10) hindurch in der Wand (9) verankert sind.

9. Gärbehälter nach einem der Ansprüche 5 bis 8, **dadurch gekennzeichnet, dass** die Verankerungselemente (14) entlang des unteren Randes der Schutzfolie (10) in einem Abstand von diesem angeordnet sind.

10. Gärbehälter nach einem der Ansprüche 5 bis 9, **dadurch gekennzeichnet, dass** mehrere der Verankerungselemente (14) in Umfangsrichtung voneinander beabstandet vertikal angeordnet sind.

11. Gärbehälter nach einem der Ansprüche 5 bis 10, **dadurch gekennzeichnet, dass** die Schutzfolie (10) einen Gewebeuntergrund (26) aus PP aufweist.

12. Gärbehälter nach Anspruch 11, **dadurch gekennzeichnet, dass** der Gewebeuntergrund (26) mit einer Schutzbeschichtung (28) aus PP versehen ist.

13. Gärbehälter nach einem Anspruch 12, **dadurch gekennzeichnet, dass** die Schutzbeschichtung (28) eine Dicke von 1,5 mm hat.

14. Gärbehälter nach einem der Ansprüche 5 bis 13, **dadurch gekennzeichnet, dass** der Kronenschutz (30) eine geringere Dicke als die Schutzfolie (10) hat.

15. Gärbehälter nach einem der Ansprüche 5 bis 14, **dadurch gekennzeichnet, dass** der Kronenschutz (30) an die Schutzfolie (10) angeschweißt ist.

## Claims

1. A method of protecting a fermentation tank (5) of a biogas plant by means of the following steps:
- arranging, on the inside of the wall (9) of the fermentation tank (5), at least one circumferentially encircling web of a protective sheet (10), which is fastened to the wall (9) by means of a plurality of anchoring elements (14);
- welding a cover (22) onto the protective sheet (10) in a media-tight manner in the region of the anchoring elements (14) and in the region of the joint (12) between the start and the end of the protective sheet (10).

2. The method according to claim 1, **characterized in that** two webs of the protective sheet (10) are mounted, the joint between the webs being sealed in a media-tight manner by means of a cover (22).

3. The method according to claim 1 or claim 2, **characterized in that** a wall coping of the wall (9) is covered by means of a coping protection (30) fitted to the protective sheet (10).

4. The method according to any of the preceding claims, **characterized in that** no concrete refurbishment is carried out before the protective sheet (10) is applied.

5. A fermentation tank (5) for a biogas plant, in particular provided with a protective sheet (10) by means of the method according to any of the preceding claims, comprising a wall (9) of concrete which is pre-damaged by aggressive media at least in a gas exchange zone, and a protective sheet (10) mounted to the wall (9) in the interior of the fermentation tank (5), the protective sheet (10) being provided at its upper edge with a coping protection (30) which extends outwards over the upper edge of the wall (9), a seal (16) being provided between the lower edge of the protective sheet (10) and the wall (9), and a plurality of anchoring elements (14) being provided by means of which the protective sheet (10) is anchored to the wall (9) of the fermentation tank (5), the anchoring elements (14) being covered in a media-tight manner by a cover (22) welded to the protective sheet (10).

6. The fermentation tank according to claim 5, **characterized in that** the protective sheet (10) consists of one circumferentially encircling web or of two circumferentially encircling webs.

7. The fermentation tank according to claim 6, **characterized in that** each web has a width on the order of 1 to 2 m.

8. The fermentation tank according to any of claims 5 to 7, **characterized in that** the anchoring elements (14) are arranged along the upper edge of the protective sheet (10) and anchored in the wall (9) through the protective sheet (10).

9. The fermentation tank according to any of claims 5 to 8, **characterized in that** the anchoring elements (14) are arranged along the lower edge of the protective sheet (10) at a distance therefrom.

10. The fermentation tank according to any of claims 5 to 9, **characterized in that** a plurality of the anchoring elements (14) are arranged vertically so as to be spaced apart from one another in the circumferential direction.

11. The fermentation tank according to any of claims 5 to 10, **characterized in that** the protective sheet (10) has a fabric base (26) made from PP.

12. The fermentation tank according to claim 11, **characterized in that** the fabric base (26) is provided with a protective coating (28) made from PP.

13. The fermentation tank according to claim 12, **characterized in that** the protective coating (28) has a thickness of 1.5 mm.

14. The fermentation tank according to any of claims 5 to 13, **characterized in that** the coping protection (30) has a thickness smaller than that of the protective sheet (10).

15. The fermentation tank according to any of claims 5 to 14, **characterized in that** the coping protection (30) is welded to the protective sheet (10).

## Revendications

1. Procédé de protection d'une cuve de fermentation (5) d'une centrale à biogaz au moyen des étapes suivantes :
- agencer, sur la face intérieure de la paroi (9) de la cuve de fermentation (5), au moins une bande circonférentielle dans le sens périphérique d'une feuille protectrice (10) qui est fixée à la paroi (9) au moyen de plusieurs éléments d'ancrage (14) ;
- souder, de manière étanche aux milieux, un recouvrement (22) sur la feuille protectrice (10) dans la zone des éléments d'ancrage (14) et dans la zone du joint (12) entre le début et la fin de la feuille protectrice (10).

2. Procédé selon la revendication 1, **caractérisé en ce que** deux bandes de la feuille protectrice (10) sont mises en place, le joint entre les bandes étant scellé de manière étanche au milieux au moyen d'un recouvrement (22).

3. Procédé selon la revendication 1 ou la revendication 2, **caractérisé en ce qu'**un couronnement de mur de la paroi (9) est recouvert au moyen d'une protection de couronnement (30) qui est agencée sur la feuille protectrice (10).

4. Procédé selon l'une des revendications précédentes, **caractérisé en ce qu'**aucun assainissement de béton n'est effectué avant l'application de la feuille protectrice (10).

5. Cuve de fermentation (5) pour une centrale à biogaz, en particulier pourvue d'une feuille protectrice (10) au moyen du procédé selon l'une des revendications précédentes, comprenant une paroi (9) en béton qui est déjà endommagée par des milieux agressifs au moins dans une zone d'échange de gaz, et une feuille protectrice (10) qui est agencée sur la paroi (9) dans l'espace intérieur de la cuve de fermentation (5), la feuille protectrice (10) étant pourvue, sur son bord supérieur, d'une protection de couronnement (30) qui s'étend vers l'extérieur au-dessus du bord supérieur de la paroi (9), un joint d'étanchéité (16) étant prévu entre le bord inférieur de la feuille protectrice (10) et la paroi (9), et plusieurs éléments d'ancrage (14) étant prévus, au moyen desquels la feuille protectrice (10) est ancrée sur la paroi (9) de la cuve de fermentation (5), les éléments d'ancrage (14) étant recouverts, de manière étanche aux milieux, d'un recouvrement (22) qui est soudé sur la feuille protectrice (10).

6. Cuve de fermentation selon la revendication 5, **caractérisée en ce que** la feuille protectrice (10) est composée d'une bande circonférentielle dans le sens périphérique ou de deux bandes circonférentielles dans le sens périphérique.

7. Cuve de fermentation selon la revendication 6, **caractérisée en ce que** chaque bande présente une largeur de l'ordre de 1 à 2 m.

8. Cuve de fermentation selon l'une des revendications 5 à 7, **caractérisée en ce que** les éléments d'ancrage (14) sont agencés le long du bord supérieur de la feuille protectrice (10) et sont ancrés dans la paroi (9) à travers la feuille protectrice (10).

9. Cuve de fermentation selon l'une des revendications 5 à 8, **caractérisée en ce que** les éléments d'ancrage (14) sont agencés le long du bord inférieur de la feuille protectrice (10) à une distance de celui-ci.

10. Cuve de fermentation selon l'une des revendications 5 à 9, **caractérisée en ce que** plusieurs des éléments d'ancrage (14) sont agencés verticalement de manière à être espacés les uns des autres dans le sens périphérique.

11. Cuve de fermentation selon l'une des revendications 5 à 10, **caractérisée en ce que** la feuille protectrice (10) présente une base en tissu (26) en PP.

12. Cuve de fermentation selon la revendication 11, **caractérisée en ce que** la base en tissu (26) est pourvue d'un revêtement protecteur (28) en PP.

13. Cuve de fermentation selon la revendication 12, **caractérisée en ce que** le revêtement protecteur (28) présente une épaisseur de 1,5 mm.

14. Cuve de fermentation selon l'une des revendications 5 à 13, **caractérisée en ce que** la protection de couronnement (30) présente une épaisseur inférieure à celle de la feuille protectrice (10).

15. Cuve de fermentation selon l'une des revendications 5 à 14, **caractérisée en ce que** la protection de couronnement (30) est soudée sur la feuille protectrice (10).
